# EUROPEAN PATENT SPECIFICATION

(11) **EP 3 416 720 B1**
(45) Date of publication and mention of the grant of the patent: **10.04.2024**
(21) Application number: 17752796.7
(22) Date of filing: 14.02.2017
(51) Int. Cl.: A61B 5/282, A61N 1/04, A61K 9/70

(54) **PRE-CONNECTABLE MEDICAL PATCH ENVELOPE**
HÜLLE FÜR VORANSCHLIESSBAREN MEDIZINISCHEN PATCH
ENVELOPPE DE TIMBRE MÉDICAL PRÉ-CONNECTABLE

(30) Priority: 18.02.2016 US 201662296637 P
(43) Date of publication of application: 26.12.2018
(73) Proprietor: Inovytec Medical Solutions Ltd., Raanana 4366507 (IL)
(72) Inventor: KANTOR, Ehud, 4531409 Hod Hasharon (IL); BARKAI, Nir, 4422111 Kfar Sava (IL)
(74) Representative: Schulz Junghans Patentanwälte PartGmbB
(86) International application number: PCT/IL2017/050188
(87) International publication number: WO 2017/141238

(56) References cited:
- WO-A1-97/24156
- WO-A1-2014/070123
- US-A- 5 827 184
- US-A- 5 958 447
- US-A1- 2006 142 831
- US-A1- 2012 253 162
- US-B1- 7 278 424
- US-B2- 7 848 824

## Description

### Field of the Invention

The invention is from the field of medical equipment. Specifically the invention relates to medical patches that are applied to the body of a patient. More specifically the invention relates to packaging for storing and applying medical electrodes and transdermal patches that are attached to the bodies of patients for different purposes.

### Background of the Invention

One of the common procedures in medical practice is attachment of electrodes to a human body either for monitoring body functions such as in ECG or EEG or for therapy such as defibrillation. Trained medical personnel know how to quickly apply these electrodes to the proper location when necessary but doing so can be quite a challenge for untrained persons, who sometimes find themselves in a situation where they are called upon to administer medical aid.

Medical emergency situations may occur in various circumstances and affect different body functions such as: trauma injuries, central nerve system injuries (e.g. stroke with apnea), cardiac conditions (e.g. coronary artery disease or cardiac arrhythmias) respiratory conditions (e.g. pulmonary embolism and chronic obstructive lung disorder, or pulmonary spastic disease), there are also systemic conditions such as anaphylactic shock, that affect multiple body functions.

Immediate medical assistance is critical to the effectiveness of life saving treatment in emergency cases. More often than not, professional medical personnel are not present at the scene of occurrence of an emergency situation, leaving the conduct of first emergency care to a lay person bystander, who is any person physically present at the scene at the time of emergency occurrence, who is capable and willing to assist, despite the lack of professional medical qualifications. The layperson care giver is therefore dependent upon lifesaving equipment which must be ultra-user friendly, as it assumes little or no medical knowledge whatsoever of the layperson care giver.

One type of critical care device that is relatively commonly available in public places is an Automated External Defibrillator (AED) device, which is a portable electronic device that applies electrical shock therapy for the treatment of cardiac arrhythmias. In the simplest of these devices, the caregiver is required to attach two electrodes to the chest of the patient. These are dual purpose electrodes that can measure ECG in one mode of operation and administer electric shock in another.

Depending on the degree of sophistication of the AED some of them require attachment of additional electrodes to measure various vital signs. An example of the latter type of device can be found in WO 2015/068164, which describes a portable system for providing decision-assisted critical care to a patient in medical emergency situations in an out of hospital setting. The system comprises components for applying oxygen therapy as well as an AED and a controller, configured to guide a caregiver in using the device.

AED is used with an unconscious patient. One of the challenges in treating a conscious patient with coronary artery disease (CAD) is the deterioration into unconscious, sudden cardiac arrest (SCA) and the need for continuously monitoring the patient's ECG with the use of additional electrodes in order to alert the care-giver and to start the AED process immediately. The time interval from SCA to defibrillation is critical and every minute saved contributes to a better outcome.

The term "transdermal patch" as used herein refers to a medicated adhesive patch that is placed on the skin to deliver a specific dose of medication through the skin and into the bloodstream. An advantage of a transdermal drug delivery route over other types of medication delivery such as oral, topical, intravenous, intramuscular, etc. is that the patch provides a controlled release of the medication into the patient.

The US patent 5,827,184 discloses a bioelectrode having a backing, a conductive layer adjacent to the backing and a layer of conductive adhesive adjacent to the conductive layer. The backing forms a sealed protective enclosure for the layer of conductive adhesive, thus when a physician is ready to apply the electrode to a patient, the electrode is converted to its second configuration, wherein the layer of conductive adhesive is exposed for contacting and adhering the bioelectrode to the body of the patient.

The US application US 2008/0210592 A1 discloses a sealed medical electrode with a conductive layer disposed on a surface of a flexible nonconductive backing sheet, wherein the electrodes are disposed with their conductive layers face-to-face and their backing sheets separably sealed together around their peripheral edges so that the backing sheets form a gas-impermeable enclosure containing conductive layers.

It is a purpose of the present invention to provide electrodes and transdermal patches for medical purposes that are packaged in a manner that facilitates their easy and fast application to a human body.

Further purposes and advantages of this invention will appear as the description proceeds.

### Summary of the Invention

Herein the word "patient" is used in its broadest sense to mean a person who receives medical care or treatment for any reason. This includes persons undergoing routine testing or undergoing a medical procedure in a doctor's office, clinic, or hospital as well as persons undergoing emergency treatment as the result of a sudden illness or traumatic injury.

In a first aspect the invention is an envelope comprised of either one sheet of material that is folded into equal parts along a central fold line with the remaining edges of the two parts sealed to each other with an adhesive to form an airtight envelope or of two sheets of material that are placed one on top of each other with their interior sides facing each other with their edges sealed to each other with an adhesive to form an airtight envelope. The envelope has at least one medical patch inside it that is attached to the interior side of each one of the parts or sheets and a layer of medical grade pressure sensitive adhesive surrounding each one of the medical patches. The pressure sensitive adhesive is applied to a part of the surface of the interior side of the two parts or two sheets to which the at least one medical patch is attached in order to attach the two parts or two sheets of material and the attached medical patches to the body of a patient. The envelope is characterized in that each medical patch is attached to its respective part or sheet such that the areas covered by the layer of medical grade pressure sensitive adhesive surrounding the medical patches do not overlap each other when the two parts or two sheets are assembled to form the envelope

In embodiments of the envelope of the invention, electrical contacts attached to the backs of the at least one medical patch poke through the part or the sheet from the interior side of the part or the sheet to the external side of the part or the sheet.

In embodiments of the envelope of the invention electrical leads are pre-connected to electrical contacts attached to the at least one medical patch during the manufacture stage.

In embodiments of the envelope of the invention the electrical contacts attached to the at least one medical patch do not poke through to the exterior side of the parts or sheets and electrical leads are pre-connected directly to the at least one medical patch and either coiled up and sealed inside the envelope to be uncoiled when the envelope is opened or pass between the edges of the two parts or two sheets to the outside with the adhesive used to seal the envelope forming an air tight seal around them.

Embodiments of the envelope of the invention comprise a controller that is pre-connected to electrical leads that are pre-connected to electrical contacts attached to the at least one medical patch. The controller is either enclosed within the envelope or is attached to the exterior of the envelope.

In embodiments of the envelope of the invention sealing of the two parts or two sheets of material together is done with a type of adhesive that will create a seal that maintains the integrity of the envelope to insure sterility of the medical patches and freshness of the medical grade adhesive layer and allows easy separation of the two parts or two sheets when required without tearing them.

In embodiments of the envelope of the invention instructions for the correct placement of the medical patches are printed on the exterior of the envelope.

In embodiments of the envelope of the invention the at least two medical patches are selected from:
a) electrodes for medical purposes or biomedical research; and
b) transdermal patches for administering medication to a patient.

In embodiments of the envelope of the invention at least one of the at least two medical patches is an electrode and the envelope comprises a layer of gel that covers each electrode. In these embodiments sealing of the two parts or two sheets of material together is done with a type of adhesive that will create a seal that maintains the integrity of the envelope to insure sterility of the at least one medical patch and freshness of the gel and the medical grade adhesive layer and allows easy separation of the two parts or two sheets when required without tearing them.

Although not encompassed by the claims, embodiments of the envelope comprise electrodes on both of the sheets of material that make up the envelope and a non-stick liner placed between the two sheets of material.

Although not encompassed by the claims, in embodiments of the envelope that comprise electrodes on both of the sheets of material the non-stick liner is present only if the areas covered with adhesive around the electrodes overlap each other when the two sheets are assembled to form the envelope.

Although not encompassed by the claims, in embodiments of the envelope that comprise a liner separating the two sheets of material the liner can be automatically removed when the two sheets of the envelope are separated from each other.

In embodiments of the envelope of the invention envelopes containing the same arrangement of at least two electrodes are supplied in different sizes with different distances between electrodes to insure correct placement of the electrodes.

In a second aspect the invention is a method of applying the at least two medical patches in the envelope of the first aspect of the invention to the skin of a patient. The method comprises: separating the two parts or two sheets of material that form the envelope, placing the parts or sheets of material with the interior side facing the patient and the at least two medical patches at the designated location on the body of the patient, and pressing on the exterior side of the sheets of material to cause the sheet and the at least one medical patch attached to the interior side of each one of the parts or sheets to adhere to the skin of the patient.

All the above and other characteristics and advantages of the invention will be further understood through the following illustrative and non-limitative description of embodiments thereof, with reference to the appended drawings.

### Brief Description of the Drawings

- Fig. 1a and Fig. 1b show schematically the electrical contacts on the exterior sides of two pieces of material that will be assembled to form an envelope configured for use with a 3-lead ECG;
- Fig. 2a and Fig. 2b show schematically the two pieces of paper of Fig. 1a and Fig. 1b with electrical leads pre-connected to the electrical contacts;
- Fig. 3a to Fig. 3b schematically show different stages in the process of opening the envelope to expose the electrodes;
- Figs. 4a to 4c, although not encompassed by the claims, schematically show how the liner separating the two pieces of paper is automatically removed when the two sides of the envelope are separated from each other; and
- Fig. 5 shows an embodiment of the envelope of the invention.

### Detailed Description of Embodiments of the Invention

In a first embodiment the medical patch is an electrode and the invention is an envelope comprising electrodes for application to the body of a patient. The electrodes, together with gel and adhesive are pre-sealed and ready to use within the envelope. The purpose of the envelope of the invention is to store medical electrodes designed for one time use in a manner that maximizes the length of time for which they can be stored and mainly to provide a simple and speedy method of application of the electrodes to a patient in time of need.

Embodiments of the envelopes of the invention can comprise any type of electrode for medical purposes, such as: EEG, ECG, ECT, defibrillation, and electrodes for electrophysiology techniques in biomedical research.

The envelopes can contain one or more electrodes depending upon the application, for example, two electrodes for defibrillation or one, three, or twelve electrodes for one, three, or twelve lead ECG respectively.

The envelope of the invention is comprised of two thin sheets of material such as paper, plastic, and paper laminated with a layer of plastic. The backs of the electrodes are attached to one side (herein the interior side) of one or both of the sheets. In an embodiment the electrical contacts to the electrodes poke through the sheet to its exterior side. Depending on the intended use, single electrodes or arrays comprising two or more electrodes can be attached to one or both of the sheets that make up the envelope.

After the electrodes are attached to the two sheets, gel is spread to cover the area over and surrounding the electrodes and a layer of medical grade pressure sensitive adhesive of a type known in the art for attaching the sheets with attached electrodes directly to the body of the patient is applied to the surface of the interior side of the sheets surrounding the area covered by gel.

Although not encompassed by the claims, if there are electrodes on both of the sheets of material that make up the envelope and if the areas covered with adhesive around the electrodes overlap each other when the two sheets are assembled to form the envelope as described below, then a non-stick liner, for example made from polyethylene film that is coated with silicon on one or both surfaces, is placed between them. In this case the liner further assists in maintaining the gel in close contact with the electrodes.

To assemble the envelope, the two sheets of material are placed one on top of each other with the interior sides facing each other and a liner between them if necessary. The edges of the two sheets are then sealed to each other with a heat or pressure sensitive adhesive to form an airtight envelope that has inside it the electrodes covered with gel and adhesive for attaching the electrodes to the body of a patient. The sealing of the two sheets of material together is done with a type of adhesive that will create a seal that, on the one hand, maintains the integrity of the envelope to insure sterility of the electrodes and freshness of the gel and adhesive layers, and on the other hand, allows easy separation of the sheets when required without tearing them.

In an embodiment of the invention the envelope can be made from one sheet of material with a fold line dividing it into two equal parts. Each part of the sheet is treated as one of the separate sheets described above. After attachment of the medical patch or electrodes and application of the gel and liner if necessary, the sheet of material is folded along the fold line and sealed along the remaining edges of the two parts.

Embodiments of the invention will now be described with reference to the figures in order to illustrate the invention. The invention is not limited to the specific embodiments shown and, as mentioned herein above, the envelope can be made of different materials and can contain one or more electrodes. In the figures, circle 10 symbolically represents the electrode covered with gel, circle 12 symbolically represents the gel covered area, circle 14 symbolically represents the area covered by adhesive, and numeral 16 identifies the adhesive used to seal the two sheets of material to form the envelope.

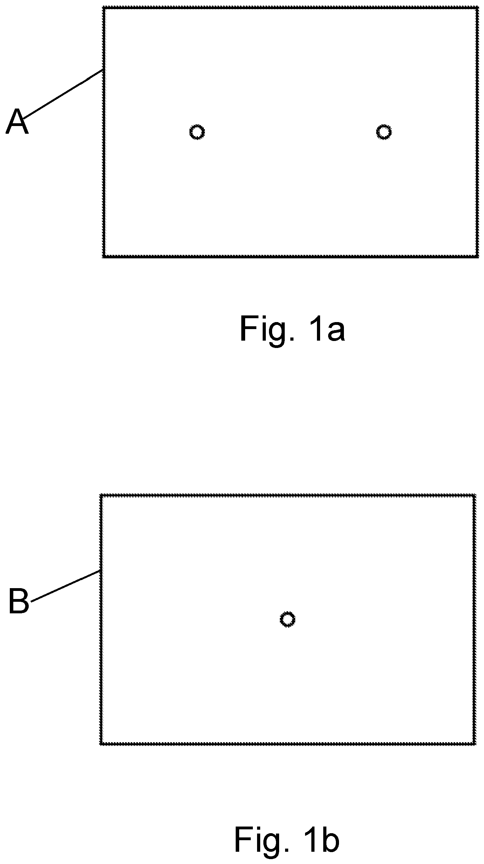

Fig. 1a and Fig. 1b show schematically the electrical contacts on the exterior sides of piece of material A, comprising two electrodes on the interior side, and B, comprising one electrode on its interior side during the manufacture stage. Pieces of material A and B will be assembled to form an envelope configured for use with a 3-lead ECG.

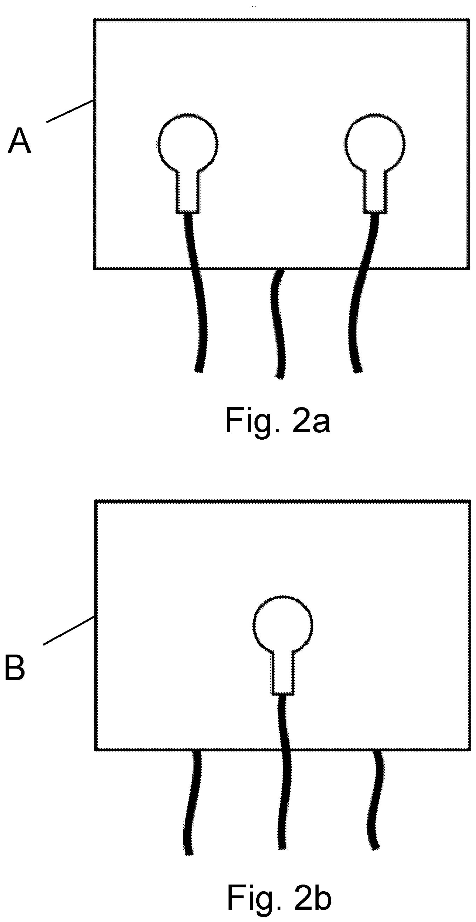

Fig. 2a and Fig. 2b show schematically the two pieces of material of Fig. 1a and Fig. 1b with electrical leads pre-connected to the electrical contacts before use. This is the favored way of supplying the envelope since with this arrangement there is no time taken or possible error in connecting the controller leads to the electrodes and the user simply has to open the envelope and invert and press it onto the body of a patient. In another embodiment the envelope is supplied without the controller electrical leads. In this pre-connectable embodiment the connection is made by a user either before or after applying the electrodes to the body of a user.

In embodiments of the envelope, the electrical contacts of the electrodes do not poke through to the exterior side of the sheets as shown in Figs. 1a and 1b. In these embodiments the electrical leads are pre-connected directly to the electrodes inside the envelope. The leads can be coiled up and sealed inside the envelope and uncoiled and connected to the external controller when the envelope is opened. Alternatively the wires of the leads can pass between the edges of the two sheets to the outside with the adhesive used to seal the envelope forming an air tight seal around them.

In embodiments of the invention a controller is enclosed within an envelope or is attached to the envelope externally. The controller can be a relatively small and inexpensive device that is pre-connected to the electrode as described herein. Such a controller can be an independent unit or a communication unit that receives instruction from an external device.

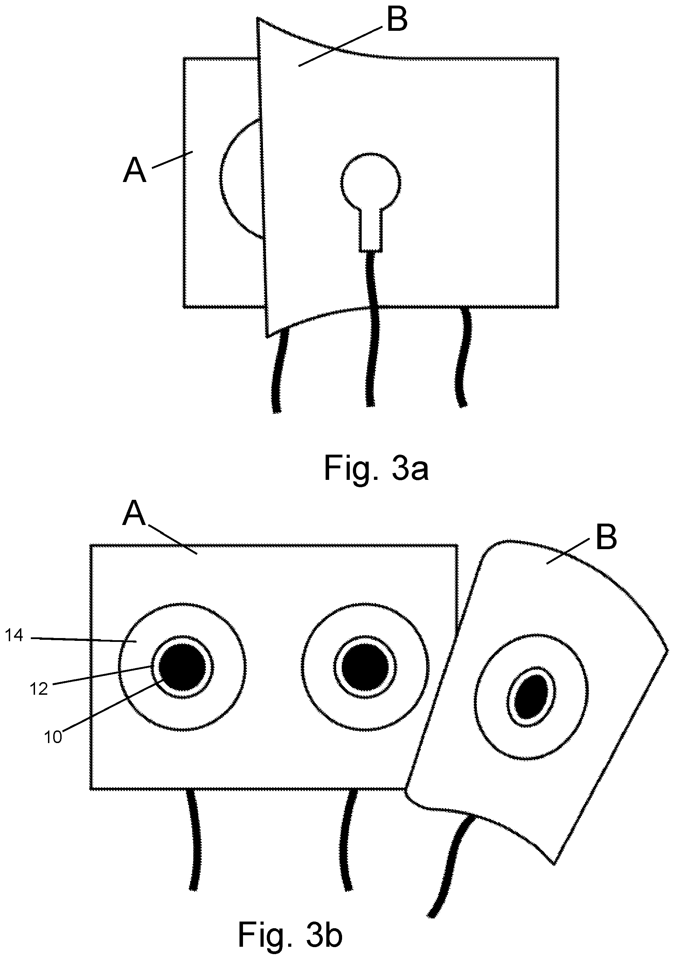

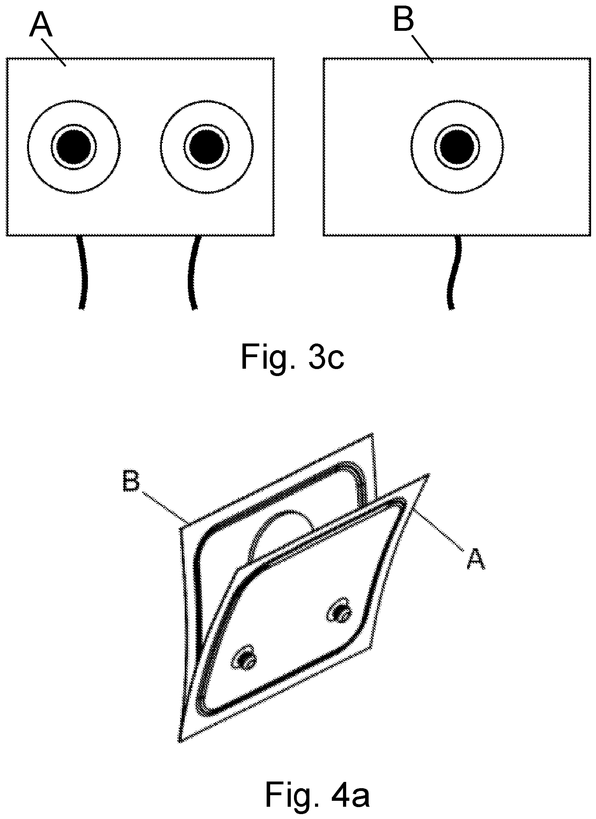

Figs. 3a to Fig. 3b schematically show different stages in the process of opening the envelope to expose the electrodes. In Fig. 3a the sealed edges of sheets A and B on one of the sides of the envelope are separated. In Fig. 3b sheets A and B are pulled apart further separating the sealed edges around the border of the envelope. In Fig. 3c sheets A and B are completely separated. They can now be inverted and pressed onto the body of a patient, where they will be held in position by the adhesive on the sheet.

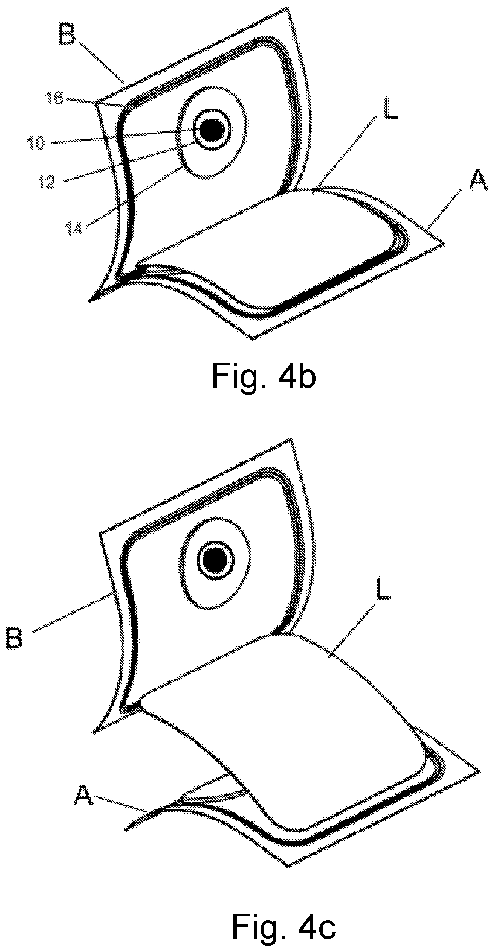

Figs. 4a to 4c, although not encompassed by the claims, schematically show how the liner separating the two pieces of material is automatically removed when the two sides of the envelope are separated from each other. In Fig. 4a the two pieces of material A and B that comprise the envelope are starting to be pulled apart. In Fig. 4b pieces of material A and B are pulled far enough apart to show liner L between them covering the adhesive covered portions of the interior surfaces. In Fig. 4c pieces of material A and B are pulled further apart. Opposite edges of liner L are attached to each of A and B. Further separation of A and B will cause L to become detached from one or both pieces of material. At this point L has automatically fallen away exposing the electrodes on both sides of the envelope and allowing the pieces of material to be attached to the patient.

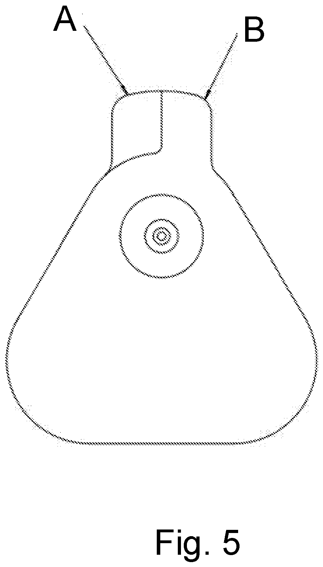

Fig. 5 shows an embodiment of the envelope of the invention. This embodiment features tabs on pieces of sheet A and B making it even easier and faster to separate them in order to attach the electrodes contained within the envelope to the body of a patient.

In order to apply the electrodes a non-experienced user or a medical professional simply has to separate the two parts of the envelope, to place the sheets of material that form the envelope with their interior sides facing the patient and the electrodes at the designated location on the body of the patient, and to press on the exterior side of the sheet of material to cause the sheet and the electrode to adhere to the skin of the patient.

Note that instructions for the correct placement of the electrodes could be printed on the exterior of the envelope or could be provided to the care-giver in another way; but, in any case, the location of the electrodes is not relevant to the present invention. It is also to be noted that application of the electrodes is simplified by the fact that at least some of them, such as those of sheet A in the figures are already arranged in arrays that have a fixed distance between them. Envelopes containing the same arrangement of electrodes are supplied in different sizes with different distances between electrodes, for example for infants, children, and small, medium or large adults, to insure correct placement of the electrodes.

ECG electrodes are typically supplied in packages of 50-100 electrodes. All electrodes in the package must be either used or discarded within a relatively short period of time, e. g. a month. This results in a considerable amount of waste for facilities that do not use large quantities of these electrodes. Therefore another aspect of the invention is that packaging individual and arrays of electrodes in the envelopes of the invention as described herein above can provide a considerable benefit in preserving the lifetime of the electrodes with attendant financial benefit.

In a second embodiment the medical patch is a transdermal patch and the invention is an envelope comprising a transdermal patch for application to the body of a patient. Non-limiting examples of medications administered by the transdermal patches contemplated by the present invention include small molecules, peptides, nucleic acids, hormones, and vitamins.

The description above of the envelopes of the first embodiment of the invention described applies also to the second embodiment *mutatis mutandis* with the electrode/s replaced with a transdermal patch.

In one embodiment, the envelope contains a transdermal patch that is integrated to one of the envelope sheets. The patch contains a medication and adhesive for attaching it to the body of a patient sealed within the envelope. Upon opening of the envelope the patch is ready to be applied to the body of the patient with no additional steps required.

In some embodiments control of the release of medication from the patch is performed by current or voltage applied by an electrical circuit. The envelope patch of the present invention is configured to be pre-connected or pre-connectable to a controller comprising a power source and the electrical circuit for managing the controlled release of the medication from the patch.

In some embodiments the controller is connected directly to the exterior of the envelope. The controller may function as an independent unit having the ability to execute the controlled release of the medication. Alternatively, the controller may have a communication function e.g. Wi-Fi, Bluetooth, and cellular. In this configuration the command to release the medication may be communicated to the controller from a remote device such as, but not limited to, a computer or a smartphone.

Although embodiments of the invention have been described by way of illustration, it will be understood that the invention may be carried out with many variations, modifications, and adaptations, without exceeding the scope of the claims.

## Claims

1. An envelope comprised of one of:
a. one sheet of material that is folded into two equal parts (A,B) along a central fold line with the remaining edges of the two parts sealed to each other with an adhesive (16) to form an airtight envelope; and
b. two sheets (A,B) of material that are placed one on top of each other with their interior sides facing each other and their edges sealed to each other with an adhesive (16) to form an airtight envelope;
the envelope containing:
i. at least one medical patch (10) attached to the interior side of each one of the parts (A,B) or sheets (A,B);
ii. a layer of medical grade pressure sensitive adhesive (14) surrounding each one of the medical patches (10); wherein the pressure sensitive adhesive (14) is applied to a part of the surface of the interior side of the two parts (A,B) or two sheets (A,B) to which the at least one medical patch is attached in order to attach the two parts (A,B) or two sheets (A,B) of material and the attached medical patches (10) to the body of a patient;
the envelope **characterized in that** each medical patch is attached to its respective part or sheet such that the areas covered by the layer of medical grade pressure sensitive adhesive surrounding the medical patches do not overlap each other when the two parts or two sheets are assembled to form the envelope.

2. The envelope of claim 1, wherein electrical contacts attached to the backs of the at least one medical patch (10) poke through the part (A,B) or the sheet (A,B) from the interior side of the part (A,B) or the sheet (A,B) to the external side of the part (A,B) or the sheet (A,B).

3. The envelope of claim 1, wherein electrical leads are pre-connected to electrical contacts attached to the at least one medical patch (10) during the manufacture stage.

4. The envelope of claim 3, wherein the electrical contacts attached to the at least one medical patch (10) do not poke through to the exterior side of the part (A,B) or the sheet (A,B) and the electrical leads are pre-connected directly to the at least one medical patch (10) and either coiled up and sealed inside the envelope to be uncoiled when the envelope is opened or pass between the edges of the two parts (A,B) or two sheets (A,B) to the outside with the adhesive (16) used to seal the envelope forming an air tight seal around them.

5. The envelope of claim 3 comprising a controller enclosed within the envelope or attached to the exterior of the envelope, the controller pre-connected to the electrical leads that are pre-connected to electrical contacts attached to the at least one medical patch (10).

6. The envelope of claim 1, wherein sealing of the two parts (A,B) or two sheets (A,B) of material together is done with a type of adhesive (16) that will create a seal that maintains the integrity of the envelope to insure sterility of the medical patches (10) and freshness of the medical grade adhesive layer (14) and allows easy separation of the two parts (A,B) or two sheets (A,B) when required without tearing them.

7. The envelope of claim 1, wherein instructions for the correct placement of the medical patches (10) are printed on the exterior of the envelope.

8. The envelope of claim 1, wherein the at least two medical patches (10) are selected from:
a) electrodes for medical purposes or biomedical research; and
b) transdermal patches for administering medication to a patient.

9. The envelope of claim 8, wherein at least one of the at least two medical patches (10) is an electrode (10) and the envelope comprises a layer of gel (12) that covers each electrode (10).

10. The envelope of claim 8, wherein sealing of the two parts (A,B) or two sheets (A,B) of material together is done with a type of adhesive (16) that will create a seal that maintains the integrity of the envelope to insure sterility of the at least one medical patch (10) and freshness of the gel (12) and the medical grade adhesive layer (14) and allows easy separation of the two parts (A,B) or two sheets (A,B) when required without tearing them.

11. The envelope of claim 8, comprising electrodes (10) on both of the parts (A,B) or the sheets (A,B) of material that make up the envelope.

12. The envelope of claim 8, wherein envelopes containing the same arrangement of at least two electrodes (10 are supplied in different sizes with different distances between electrodes (10) to insure correct placement of the electrodes (10).

13. A method of applying the at least two medical patches (10) in the envelope of claim 1 to the skin of a patient, comprising: separating the two parts (A,B) or two sheets (A,B) of material that form the envelope, placing the parts (A,B) or sheets (A,B) of material with the interior side facing the patient and the at least two medical patches (10) at designated locations on the body of the patient, and pressing on the exterior side of the parts (A,B) or sheets (A,B) of material to cause the part (A,B) or sheet (A,B) and the at least one medical patch (10) attached to the interior side of each one of the parts (A,B) or sheets (A,B) to adhere to the skin of the patient.

14. The envelope according to any of the claims 1-12 wherein the envelope comprises one of:
a. one sheet of material that is folded into two equal parts (A,B) along a central fold line with the remaining three edges of the two parts sealed to each other with an adhesive (16) to form an airtight envelope; and
b. two sheets (A,B) of material that are placed one on top of each other with their interior sides facing each other and their four edges sealed to each other with an adhesive (16) to form an airtight

## Patentansprüche

1. Hülle, bestehend aus einem von:
a. einem Materialbogen, der entlang einer zentralen Faltlinie in zwei gleiche Teile (A, B) gefaltet ist, wobei die verbleibenden Kanten der beiden Teile mit einem Klebstoff (16) miteinander versiegelt sind, um eine luftdichte Hülle auszubilden; und
b. zwei Bögen (A, B) aus Material, die so übereinander angeordnet sind, dass ihre Innenseiten einander zugewandt sind und ihre Kanten mit einem Klebstoff (16) miteinander versiegelt sind, um eine luftdichte Hülle auszubilden;
wobei die Hülle Folgendes enthält:
i. mindestens einen medizinischen Patch (10), der an der Innenseite eines jeden der Teile (A, B) oder Bögen (A, B) angebracht ist;
ii. eine Schicht aus druckempfindlichem Klebstoff (14) medizinischer Qualität, die jedes der medizinischen Patches (10) umgibt; wobei der druckempfindliche Klebstoff (14) auf einen Teil der Fläche der Innenseite der zwei Teile (A, B) oder zwei Bögen (A, B), an denen der mindestens eine medizinische Patch angebracht ist, angewendet wird, um die zwei Teile (A, B) oder zwei Bögen (A, B) aus Material und die angebrachten medizinischen Patches (10) am Körper eines Patienten anzubringen;
wobei die Hülle **dadurch gekennzeichnet ist, dass** jeder medizinische Patch an seinem jeweiligen Teil oder Bogen so angebracht ist, dass sich die Bereiche, die von der Schicht aus druckempfindlichem Klebstoff medizinischer Qualität, die die medizinischen Patches umgibt, bedeckt sind, nicht gegenseitig überlappen, wenn die beiden Teile oder die beiden Bögen zusammengefügt werden, um die Hülle auszubilden.

2. Hülle nach Anspruch 1, wobei an den Rückseiten des mindestens einen medizinischen Patches (10) angebrachte elektrische Kontakte das Teil (A, B) oder den Bogen (A, B) von der Innenseite des Teils (A, B) oder des Bogens (A, B) zur Außenseite des Teils (A, B) oder des Bogens (A, B) durchstechen.

3. Hülle nach Anspruch 1, wobei elektrische Leitungen mit elektrischen Kontakten, die an dem mindestens einen medizinischen Patch (10) angebracht sind, während der Herstellungsphase vorverbunden werden.

4. Hülle nach Anspruch 3, wobei die elektrischen Kontakte, die an dem mindestens einen medizinischen Patch (10) angebracht sind, nicht die Außenseite des Teils (A, B) oder des Bogens (A, B) durchstechen und die elektrischen Leitungen direkt mit dem mindestens einen medizinischen Patch (10) vorverbunden sind und entweder aufgerollt und im Inneren der Hülle versiegelt sind, um beim Öffnen der Hülle abgerollt zu werden, oder zwischen den Kanten der beiden Teile (A, B) oder der beiden Bögen (A, B) nach außen hindurchgehen, wobei der zum Versiegeln der Hülle verwendete Klebstoff (16) eine luftdichte Versiegelung um sie herum bildet.

5. Hülle nach Anspruch 3, umfassend eine Steuerung, die in der Hülle eingeschlossen oder an der Außenseite der Hülle angebracht ist, wobei die Steuerung mit den elektrischen Leitungen vorverbunden ist, die mit elektrischen Kontakten vorverbunden sind, die an dem mindestens einen medizinischen Patch (10) angebracht sind.

6. Hülle nach Anspruch 1, wobei das Versiegeln der beiden Teile (A, B) oder der beiden Bögen (A, B) aus Material mit einer Art von Klebstoff (16) erfolgt, der eine Versiegelung erzeugt, die die Integrität der Hülle aufrechterhält, um die Sterilität der medizinischen Patches (10) und die Frische der Klebeschicht (14) medizinischer Qualität zu gewährleisten, und eine einfache Trennung der beiden Teile (A, B) oder der beiden Bögen (A, B) ermöglicht, wenn dies erforderlich ist, ohne sie zu zerreißen.

7. Hülle nach Anspruch 1, wobei auf der Außenseite der Hülle Anweisungen für die korrekte Platzierung der medizinischen Patches (10) aufgedruckt sind.

8. Hülle nach Anspruch 1, wobei die mindestens zwei medizinischen Patches (10) ausgewählt sind aus:
a) Elektroden für medizinische Zwecke oder biomedizinische Forschung; und
b) transdermale Patches zur Verabreichung von Medikamenten an einen Patienten.

9. Hülle nach Anspruch 8, wobei mindestens eines der mindestens zwei medizinischen Patches (10) eine Elektrode (10) ist und die Hülle eine Gelschicht (12) umfasst, die jede Elektrode (10) bedeckt.

10. Hülle nach Anspruch 8, wobei das Versiegeln der beiden Teile (A, B) oder der beiden Bögen (A, B) aus Material mit einer Art von Klebstoff (16) erfolgt, der eine Versiegelung erzeugt, die die Integrität der Hülle aufrechterhält, um die Sterilität des mindestens einen medizinischen Patches (10) und die Frische des Gels (12) und der Klebeschicht (14) medizinischer Qualität zu gewährleisten, und eine einfache Trennung der beiden Teile (A, B) oder der beiden Bögen (A, B) ermöglicht, wenn dies erforderlich ist, ohne sie zu zerreißen.

11. Hülle nach Anspruch 8, umfassend Elektroden (10) auf den beiden Teilen (A, B) oder den Bögen (A, B) aus Material, aus denen die Hülle besteht.

12. Hülle nach Anspruch 8, wobei Umschläge, die die gleiche Anordnung von mindestens zwei Elektroden (10) enthalten, in unterschiedlichen Größen mit unterschiedlichen Abständen zwischen den Elektroden (10) geliefert werden, um eine korrekte Platzierung der Elektroden (10) zu gewährleisten.

13. Verfahren zum Anwenden der mindestens zwei medizinischen Patches (10) in der Hülle nach Anspruch 1 auf der Haut eines Patienten, umfassend: Trennen der beiden Teile (A, B) oder der beiden Bögen (A, B) aus Material, welche die Hülle ausbilden, Platzieren der Teile (A, B) oder Bögen (A, B) aus Material, wobei die Innenseite dem Patienten zugewandt ist, und der mindestens zwei medizinischen Patches (10) an bestimmten Stellen auf dem Körper des Patienten, und Drücken auf die Außenseite der Teile (A, B) oder Bögen (A, B) aus Material, um zu bewirken, dass das Teil (A, B) oder der Bogen (A, B) und der mindestens eine medizinische Patch (10), das an der Innenseite von jedem der Teile (A, B) oder Bögen (A, B) angebracht ist, an der Haut des Patienten haften.

14. Hülle nach einem der Ansprüche 1 bis 12,
wobei die Hülle eines umfasst von:
a. einem Materialbogen, der entlang einer zentralen Faltlinie in zwei gleiche Teile (A, B) gefaltet ist, wobei die verbleibenden drei Kanten der beiden Teile mit einem Klebstoff (16) miteinander versiegelt sind, um eine luftdichte Hülle auszubilden; und
b. zwei Bögen (A, B) aus Material, die so übereinander angeordnet sind, dass ihre Innenseiten einander zugewandt sind und ihre vier Kanten mit einem Klebstoff (16) miteinander versiegelt sind, um eine luftdichte Hülle auszubilden;

## Revendications

1. Enveloppe constituée d'un élément parmi :
a. une feuille de matériau qui est pliée en deux parties égales (A, B) le long d'une ligne de pliage centrale avec les bords restants des deux parties scellés l'un à l'autre avec un adhésif (16) pour former une enveloppe étanche à l'air ; et
b. deux feuilles (A, B) de matériau qui sont placées l'une au-dessus de l'autre avec leurs côtés intérieurs tournés l'un vers l'autre et leurs bords scellés l'un à l'autre avec un adhésif (16) pour former une enveloppe étanche à l'air ;
l'enveloppe contenant :
i. au moins un timbre médical (10) relié au côté intérieur de chacune des parties A, B) ou feuilles (A, B) ;
ii. une couche d'adhésif sensible à la pression de qualité médicale (14) entourant chacun des timbres médicaux (10) ; dans laquelle l'adhésif sensible à la pression (14) est appliqué sur une partie de la surface du côté intérieur des deux parties (A, B) ou deux feuilles (A, B) à laquelle l'au moins un timbre médical est relié afin de relier les deux parties (A, B) ou deux feuilles (A, B) de matériau et les timbres médicaux reliés (10) au corps d'un patient ;
l'enveloppe étant **caractérisée en ce que** chaque timbre médical est relié à sa partie ou feuille respective de sorte que les zones recouvertes par la couche d'adhésif sensible à la pression de qualité médicale entourant les timbres médicaux ne se chevauchent pas lorsque les deux parties ou deux feuilles sont assemblées pour former l'enveloppe.

2. Enveloppe selon la revendication 1, dans laquelle des contacts électriques reliés aux dos de l'au moins un timbre médical (10) passent à travers la partie (A, B) ou la feuille (A, B) du côté intérieur de la partie (A, B) ou la feuille (A, B) au côté externe de la partie (A, B) ou la feuille (A, B).

3. Enveloppe selon la revendication 1, dans laquelle des fils électriques sont préconnectés à des contacts électriques reliés à l'au moins un timbre médical (10) au cours de l'étape de fabrication.

4. Enveloppe selon la revendication 3, dans laquelle les contacts électriques reliés à l'au moins un timbre médical (10) ne passent pas à travers vers le côté extérieur de la partie (A, B) ou la feuille (A, B), et les fils électriques sont directement préconnectés à l'au moins un timbre médical (10) et soit enroulés et scellés à l'intérieur de l'enveloppe pour être déroulés lorsque l'enveloppe est ouverte, soit passent entre les bords des deux parties (A, B) ou deux feuilles (A, B) vers l'extérieur avec l'adhésif (16) utilisé pour sceller l'enveloppe formant un joint étanche à l'air autour d'eux.

5. Enveloppe selon la revendication 3, comprenant un contrôleur renfermé au sein de l'enveloppe ou relié à l'extérieur de l'enveloppe, le contrôleur étant préconnecté aux fils électriques qui sont préconnectés à des contacts électriques reliés à l'au moins un timbre médical (10).

6. Enveloppe selon la revendication 1, dans laquelle le scellement des deux parties (A, B) ou deux feuilles (A, B) de matériau ensemble est réalisé avec un type d'adhésif (16) qui créera un joint qui maintient l'intégrité de l'enveloppe pour garantir la stérilité des timbres médicaux (10) et la fraîcheur de la couche d'adhésif de qualité médicale (14), et permet une séparation facile des deux parties (A, B) ou deux feuilles (A, B) lorsqu'elle est requise sans les déchirer.

7. Enveloppe selon la revendication 1, dans laquelle des instructions pour le placement correct des timbres médicaux (10) sont imprimées sur l'extérieur de l'enveloppe.

8. Enveloppe selon la revendication 1, dans laquelle les au moins deux timbres médicaux (10) sont sélectionnés parmi :
a) des électrodes à des fins médicales ou pour la recherche biomédicale ; et
b) des timbres transdermiques pour l'administration de médicament à un patient.

9. Enveloppe selon la revendication 8, dans laquelle au moins un des au moins deux timbres médicaux (10) est une électrode (10) et l'enveloppe comprend une couche de gel (12) qui recouvre chaque électrode (10).

10. Enveloppe selon la revendication 8, dans laquelle le scellement des deux parties (A, B) ou deux feuilles (A, B) de matériau ensemble est réalisé avec un type d'adhésif (16) qui créera un joint qui maintient l'intégrité de l'enveloppe pour garantir la stérilité de l'au moins un timbre médical (10) et la fraîcheur du gel (12) et de la couche d'adhésif de qualité médicale (14), et permet une séparation facile des deux parties (A, B) ou deux feuilles (A, B) lorsqu'elle est requise sans les déchirer.

11. Enveloppe selon la revendication 8, comprenant des électrodes (10) sur les deux parties (A, B) ou les deux feuilles (A, B) de matériau qui constituent l'enveloppe.

12. Enveloppe selon la revendication 8, dans laquelle des enveloppes contenant le même agencement d'au moins deux électrodes (10) sont fournies en différentes tailles avec différentes distances entre les électrodes (10) pour garantir le placement correct des électrodes (10).

13. Procédé d'application des au moins deux timbres médicaux (10) dans l'enveloppe selon la revendication 1 sur la peau d'un patient, comprenant : séparer les deux parties (A, B) ou deux feuilles (A, B) de matériau qui forment l'enveloppe, placer les parties (A, B) ou feuilles (A, B) de matériau avec le côté intérieur tourné vers le patient et les au moins deux timbres médicaux (10) à des emplacements désignés sur le corps du patient, et appuyer sur le côté extérieur des parties (A, B) ou feuilles (A, B) de matériau pour amener la partie (A, B) ou feuille (A, B) et l'au moins un timbre médical (10) relié au côté intérieur de chacune des parties (A, B) ou feuilles (A, B) à adhérer à la peau du patient.

14. Enveloppe selon l'une quelconque des revendications 1 à 12, dans laquelle
l'enveloppe comprend un élément parmi :
a. une feuille de matériau qui est pliée en deux parties égales (A, B) le long d'une ligne de pliage centrale avec les trois bords restants des deux parties scellés l'un à l'autre avec un adhésif (16) pour former une enveloppe étanche à l'air ; et
b. deux feuilles (A, B) de matériau qui sont placées l'une au-dessus de l'autre avec leurs côtés intérieurs tournés l'un vers l'autre et leurs quatre bords scellés l'un à l'autre avec un adhésif (16) pour former une enveloppe étanche à l'air.
